(19) Europäisches Patentamt
European Patent Office
Office européen des brevets

(11) Publication number: **0 386 289 A1**

# (12) EUROPEAN PATENT APPLICATION

(21) Application number: 89104023.0

(22) Date of filing: 07.03.89

(51) Int. Cl.⁵: C12N 15/00, C12P 21/02, C12N 5/00, C12P 21/00, //(C12P21/00,C12R1:91)

The title of the invention has been amended (Guidelines for Examination in the EPO, A-III, 7.3).

Claims 11 and 12 are deemed to be abandoned due to non-payment of the claims fees (Rule 31 (2) EPC).

(43) Date of publication of application: 12.09.90 Bulletin 90/37

(84) Designated Contracting States: GB

(71) Applicant: Taniguchi, Tadatsugu, Dr.
Institute for Molecular and Cellular Biology
Osaka University 1-3, Yamadaoka
Suita-shi Osaka 565(JP)

Applicant: Miyasaka, Masayuki, Dr.
Department of Immunology The Tokyo
Metropolitan Institute of Medical Science
Bunkyo-ku Tokyo 113(JP)

(72) Inventor: Taniguchi,Tadatsugu,Prof.
Mihogaoka 19,A-207
Ibaraki-shi,Osaka 567(JP)
Inventor: Hatakeyama,Masanori,Dr.
21-E-603,Kashikiriyama
Suita-shi,Osaka 563(JP)
Inventor: Minamoto,Sejiro,Dr.
1621-1-301,Ohaza-aomadai
Minou-shi,Osaka 562(JP)
Inventor: Kono,Takeshi
1-8-20-301,Teshimakita
Ikeda-shi,Osaka 563(JP)
Inventor: Doi,Takeshi
1-28-6,Hana-koganei-minamimachi
Kodaira-shi,Tokyo 187(JP)
Inventor: Miyasaka,Masayuki,Dr.
636-104,Mimuro
Wrawa-shi,Saitama 336(JP)
Inventor: Tsudo,Mitsuru,Dr.
3-37-1-204,Narimasu
Itabashi-ku,Tokyo 175(JP)

(74) Representative: Laudien, Dieter, Dr. et al
Boehringer Ingelheim Zentrale GmbH ZA
Patente Postfach 200
D-6507 Ingelheim am Rhein(DE)

## (54) Recombinant interleukin-2 receptor.

(57) Recombinant IL-21Rβ chain or portions thereof, cDNA coding therefore, vectors containing said cDNA, hosts transfected by said vectors, and monoclonal antibodies to said recombinant IL-2Rβ or portions thereof.



Xerox Copy Centre

# Recombinant Protein Receptor

This invention relates to receptors for interleukin-2, more particularly to the β-chain of the receptor, and to cDNA coding for the β-chain or parts thereof, vectors containing cDNA inserts coding for the β-chain, hosts transformed by such vectors and the cultivation of such hosts to produce the said β-chain.

Ample evidence has been accumulated that cytokines, a class of soluble mediators involved in cell-to-cell "communications", are essential in the regulation of the immune system. It has been known that cytokines induce proliferation, differentiation and activation of target cells through interaction with specific cell surface receptor(s). Interleukin-2 (IL-2), previously defined as T cell growth factor (1), is one of the best characterized cytokines, known to play a pivotal role in the antigen-specific clonal proliferation of T lymphocytes (T cells) (2). IL-2 also appears to act on other cells of the immune system such as immature thymocytes (3), B lymphocytes (B cells) (4), macrophages (5), natural killer cells (NK cells) (6), and lymphokine-activated killer cells (LAK cells) (7). These multifunctional properties of IL-2 have opened now possibilities in the formulation of immunotherapies such as adoptive immunotherapy (8). More recently, IL-2 has been shown to function also on neural cells such as oligodendrocytes (9), suggesting a possible involvement of this cytokine in the central nervous system. Despite extensive studies on the IL-2 system in the context of basic and clinical immunology, information has been limited on the molecular mechanism(s) underlying the IL-2-mediated signal transduction (10).

The IL-2 receptor (IL 2R) is known to be unique in that it is present in three forms: high-, intermediate- and low-affinity forms with respect to its binding ability to IL-2, and respective dissociation constants (Kds) of $10^{-11}$M, $10^{-9}$M and $10^{-8}$M (11, 12). Following the characterization of IL-2Rα chain (Tac antigen, p55) (13), it became evident that the α chain constitutes solely the low-affinity form and it is not functional per se in IL-2 internalization and signal transduction, unless associated with another specific membrane component(s) of lymphoid cells (14, 15). Subsequently, the lymphoid membrane component was identified to be a novel receptor chain, termed β chain (or p70-75) (12, 16, 17). In fact, experimental evidence has suggested that the IL-2Rβ chain per se constitutes the intermediate-affinity form (12). In addition, its association with the IL-2Rα chain results in the high-affinity form of the receptor (12, 16, 17). Expression studies using wild type and mutated IL-2Rα chain cDNAS strongly support the notion that the IL-2Rβ chain but not the IL-2Rα chain possesses a domain(s) responsible in driving the intracellular signal transduction pathway(s) (18). There exists therefore a need to obtain IL-2β chain in amounts which will enable its structure and function to be elucidated, this being an essential step in gaining further insight into the molecular basis of the high-affinity IL-2R as well as on the mechanism of signal transduction operating in IL-2 responsive cells. To this end we describe below cDNA coding for the IL-2β chain or parts thereof whereby insertion of said cDNA in a suitable vector and expression thereof in an appropriate host will enable recombinant and large scale production of protein corresponding to the IL-2β chain or parts thereof.

## Isolation and analysis of the cDNA clones

In isolating the cDNA clones, we applied an expression cloning strategy by using the monoclonal antibodies, Mik-β1 and Mik-β2 (19), both of which have been raised against the IL-2Rβ chain found on the human leukemic cell line (YT (20). The monoclonal antibodies Mik-β1 and Mik-β2 are both deposited at Fermentation Research Institute, Agency of Industrial Science and Technology, Japan. The deposit numbers for Mik-β1 and Mik-β2 are, 10453 and 10454 (1988), respectively; they are also described in Japanese Patent Application No. 298742 (1988).

A few sets of cDNA libraries were prepared by using the poly(A)$^+$-RNA from YT cells according to standard procedures. cDNA libraries were prepared with cDM8 vector according to published procedures (21), except using random primer (Amersham) or oligo (dT) primer as mentioned below. The plasmid DNA representing $5.6x10^6$ independent colonies were prepared by the standard procedure and one mg of DNA were used for the first DNA transfection. Actually, the DNA was divided into 100 tubes (therefore each tube contained 10 μg of DNA) and they were each transfected into $3.5x10^5$ monkey COS cells in a tissue culture dish (60 mm polystyrene dish, Corning). The transfection was done using the standard DEAE dextran procedures. The transfected COS cells were then treated with the cocktail of Mik-β1 and -β2 antibodies (400-fold diluted ascites for each antibody) and subjected to the standard panning procedure. The dish used for the panning was FALCON 60 mm dish, coated with anti-mouse IgG as described previously (ref. 21). In this first round of panning, 100 IgG-coated dishes were used. After the panning, Hirt extract was prepared by the standard procedure (ref. 21) and the recovered plasmids were introduced into E.coli by the method described in ref. 21. By this procedure 3.7x10$^{6}$ colonies were obtained. Those bacterial colonies were fused with COS cells by the standard protoplast fusion procedures (ref. 21). In these fusion experiments, 26 Corning dishes each containing 5x10$^{5}$ COS cells were used. After the fusion, the COS cells were subjected to panning as described above and Hirt extract was prepared. 32,000 bacterial colonies were obtained from the Hirt extract. The fusion, panning procedures were repeated again and 32,000 bacterial colonies were obtained from the subsequent Hirt extract. The same procedure were repeated once again, obtaining 28,000 bacterial colonies (in the meantime, there should be a dramatic enrichment of the objective clones). The same procedures were repeated once again and 6,000 colonies were obtained. From these colonies, 30 colonies were picked up randomly and the cDNA inserts were analysed. Of them, only 7 colonies contained plasmids from which cDNA inserts can be excised by restriction enzyme XhoI. The vector drived XhoI sites are located at the both side of the cDNA and all other plasmids had lost such cleavage sites due to the DNA rearrangements; in fact, all of them were much smaller in size than the original vector. Thus they were considered to be non-specific products. On the other hand, all of the 7 colonies were derived from the same mRNA, as confirmed by the conventional restriction enzyme cleavage analysis and DNA blot analysis. Of them, one plasmid, termed pIL-2Rβ30 contained longer cDNA than other 6 plasmids which were turned out to be identical to each other (designated gTT-2Rβ9).

In this procedure, therefore, we isolated two independent cDNA clones, pIL-2Rβ9 and pIL-2β30; each of the expression products specifically reacted with the antibodies. The two clones contained cDNA inserts of 1.3Kb and 2.3Kb, respectively, and cross-hybridized with each other. Subsequent sequence analysis of the cDNAs revealed that they represent the same mRNA. In fact, RNA blotting analysis revealed that the mRNA is approximately 4Kb in size (see below). Subsequently, we screened other YT cDNA libraries by using the cloned cDNAs as probes, and several independent cDNA clones which together cover the entire mRNA for the IL-2Rβ chain were isolated. Thus pIL-2Rβ6 and pIL-2Rβ19 were obtained by screening the cDNA libraries with the pIL-2Rβ9 cDNA insert in the probe.

The above mentioned plasmids containing cDNA coding for IL-2β sequences have been deposited in strain E.coli MC 1061/P3 on March 2, 1989 at the Fermentation Research Institute according to the Budapest Treaty under the following accession numbers:

| Plasmid | Accession No. |
|---|---|
| pIL-2Rβ6 | FERM BP-2312 |
| pIL-2Rβ9 | FERM BP-2313 |
| pIL-2Rβ19 | FERM BP-2314 |
| pIL-2Rβ30 | FERM BP-2315 |

The complete nucleotide sequences of four of the cloned cDNAs were determined (Fig. 1).

Fig. 1 shows the structure of the human IL-2Rβ chain cDNA. Fig. 1a is a schematic representation of the mRNA as deduced from the cloned cDNAs. Dotted, hatched, open and closed rectangles represent respectively the signal sequence, the extracellular, the transmembrane and the cytoplasmic regions of the mRNA are shown below. Fig. 1b shows the nucleotide and amino acid sequences of the human IL-2Rβ chain cDNA. The sequence was deduced following the complete DNA sequence analysis of the above described cDNA clones. Nucleotides are numbered on the right margin and amino acids are numbered on the left margin. Clones pIL-2Rβ19 and pIL-2Rβ6 each contained G-A mutation at nucleotide residues 425 and 1531, respectively. Thus pIL-2Rβ6 cDNA acquired a TAG triplet in the cytoplasmic region. It is thought to be an error in reverse transcription, since all other clones, pIL-2Rβ30, pIL-2Rβ19 and pIL-2Rβ16 (28), have TGG triplet at that position. The first underlined 26 amino acid residues represent the signal sequence as predicted by the consensus sequence (22). The 25 transmembrane amino acid residues are marked with a thick underlining. The cysteine residues are boxed. The potential N-glycosylation sites are underlined twice. The possible poly-adenylation signals are shown by open rectangle. RNA was prepared from the NK-like human lymphoid cell line, YT, and cDNA libraries were prepared with CDM8 vector according to published procedures (21), except using either random primers (Amersham) (for pIL-2Rβ6, 9 and 30), or oligo (dT) primer (for pIL-2Rβ19). Screening of the cDNA libraries by a cocktail of anti-IL-2Rβ monoclonal antibodies, Mik-βl and Mik-β2, was carried out as described previously (21). Nucleotide sequences were determined by a combination of dideoxy chain termination and chemical cleavage methods.

As shown in Fig. 1, the cDNA contains a large open reading frame that encodes a protein consisting of 551 amino acids. No significant homology with other known proteins was found in the Protein Sequence Database (National Biomedical Research Foundation, Washington, D.C.) or with sequences published more recently. Unlike many of other cytokine receptors, it appears that IL-2Rα and Il-2Rβ chains do not belong to the immunoglobulin superfamily. From the deduced structure of the protein, the N-terminal 26 amino acids is considered to represent the signal sequence (Fig. 1 and 2) (22). Thus the natured form of the IL-2Rβ chain consists of 525 amino acids with a calculated M.W. of 58.358. As shown in Fig. 1, the receptor molecule consists of an extracellular region consisting of 214 amino acids. This region contains 8 cysteine residues of which 5 residues are found in the N-terminal half and they are interspaced rather periodically by 9-12 amino acids. It is likely that disulfide linkages between the cysteine residues impart a stable configuration for ligand binding. In fact, abundance of cysteine residues seems to be one of the common features of the ligand binding domain of many receptors (23). It may be worth noting that the predicted number of amino acids (a.a.) within the extracellular region of the Il-2Rβ chain (214 a.a.) is almost comparable in number to that of the IL-2Rα chain (219 a.a.). Such size similarity may be significant in considering the conformation of the heterodimeric receptor complex that is quite unique for this receptor; as both α and β chains individually interact with distinct sites of the same IL-2 molecule (24).

A hydrophobic stretch of 25 amino acids spanning from the 215 to 239 amino acid residues appears to constitute the membrane spanning region of the receptor (Fig. 1 and 2).

Fig. 2 is a hydropathy plot analysis of deduced human IL-2Rα and Il-2Rβ chain precursor structures. The analysis was carried out according to Kyte and Doolittle (38). SG and TM each represents signal sequence and transmembrane sequence, respectively.

The cytoplasmic region of the β chain consists of 286 a.a. and it is far larger than that of the α chain, which is only 13 a.a. long. The consensus sequences of tyrosine kinase (Gly-x-Gly-x-x-Gly) (25) are absent in the β chain. However, the presence of a triplet, Ala-Pro-Glu (293-295) may be noted; this has been asserted to be the consensus motif for a catalytic domain of some protein kinases (25). The possibility of the cytoplasmic region of the β chain having a protein kinase activity has yet to be tested. The primary structure of this region revealed yet another interesting feature; a rather strong bias for certain characteristic amino acids. This region is rich in proline (42/286) and serine (30/286) residues. Interestingly, the "proline rich" structure has also been demonstrated in the cytoplasmic region of CD2, a T cell membrance antigen involved in the activation pathway of T cells (26). The proline-rich structure may impart a non-globular conformation to this region that may be important in coupling of the receptor molecule with other signal transducer(s). The predominant serine residues may be the major target for phosphorylation, which could also modulate the receptor function (27). In addition, the cytoplasmic region is notably biased for negatively charged amino acids. In fact, this region contains 40 such amino acids (i.e. glutamic and aspartic acids), whereas only 18 amino acids account for the positively charged residues (i.e. lysine and arginine). Such a bias is particularly notable in the middle portion (a.a. 345-390) of the cytoplasmic region. Thus, the cytoplasmic region of the β chain may be quite acidic. Taken together some if not all of these unique characteristics may be responsible in driving further the downstream signal transduction pathway(s). The receptor protein contains 5 potential sites for N-linked glycosylation (Fig. 1), in which 4 are located in the extracellular region. Such a posttranslational modification may account for the difference between the M.W. of the estimated mature (70-75kD) and the calculated (58kD) protein molecules. Hydropathy plot analysis of the α and β chains revealed the presence of hydrophilic regions just adjacent to the cell membrance in the both chains (Fig. 2) These regions may play a role in the non-covalent intramolecular association between the two chains.

According to a broad aspect of the present invention therefore we provide a recombinant cDNA coding for the IL-2Rβ chain.

Preferably the cDNA is defined by a structural gene having formula:

GCAGCCAGAGCTCAGCAGGGCCCTGGAGAGATGG
CCACGGTCCCAGCACCGGGGAGGACTGGAGAGCGCGCTGCCACCGCCCC
ATGTCTCAGCCAGGGCTTCCTTCCTCGGCTCCACCCTGTGGATGTA ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG
AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG
ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT

GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG
GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA
ATC CAC TTG GTG TAG ACAGATGGCCAGGGTGGGAGGCAGGCAGCT
GCCTGCTCTGCGCCGAGCCTCAGAAGGACCCTGTTGAGGGTCCTCAGTCCA
CTGCTGAGGACACTCAGTGTCCAGTTGCAGCTGGACTTCTCCACCCGGATG
GCCCCCACCCAGTCCTGCACACTTGGTCCATCCATTTCCAAACCTCCACTG
CTGCTCCCGGGTCCTGCTGCCCGAGCCAGGAACTGTGTGTGTTGCAGGGGG
GCAGTAACTCCCCAACTCCCTCGTTAATCACAGGATCCCACGAATTTAGGC
TCAGAAGCATCGCTCCTCTCCAGCCCTGCAGCTATTCACCAATATCAGTCC
TCGCGGCTCTCCAGGGCTCCCTGCCCTGACCTCTTCCCTGGGTTTTCTGCC
CCAGCCTCCTCCTTCCCTCCCCTCCCCGTCCACAGGGCAGCCTGAGCGTGC
TTTCCAAAACCCAAATATGGCCACGCTCCCCCTCGGTTCAAAACCTTGCAC
AGGTCCCACTGCCCTCAGCCCCACTTCTCAGCCTGGTACTTGTACCTCCGG
TGTCGTGTGGGGACATCCCCTTCTGCAATCCTCCCTACCGTCCTCCCGAGC
CACTCAGAGCTCCCTCACACCCCCTCTGTTGCACATGCTATTCCCTGGGGC
TGCTGTGCGCTCCCCCTCATCTAGGTGACAAACTTCCCTGACTCTTCAAGT
GCCGGTTTTGCTTCTCCTGGAGGGAAGCACTGCCTCCCTTAATCTGCCAGA
AACTTCTAGCGTCAGTGCTGGAGGGAGAAGCTGTCAGGGACCCAGGGCGCC
TGGAGAAAGAGGCCCTGTTACTATTCCTTTGGGATCTCTGAGGCCTCAGAG
TGCTTGGCTGCTGTATCTTTAATGCTGGGGCCCAAGTAAGGGCACAGATCC
CCCCGACAAAGTGGATGCCTGCTGCATCTTCCCACAGTGGCTTCACAGACC
CACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACT
GCCCCCTCCACTGGAGGGGTCTCCTCTGCAGCTCAGGGACTGGCACACTGG
CCTCCAGAAGGGCAGCTCCACAGGGCAGGGCCTCATTATTTTTCACTGCCC
CAGACACAGTGCCCAACACCCCGTCGTATACCCTGGATGAACGAATTAATT
ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC

AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT

The present invention also includes cDNA coding for portions of the complete sequence of the IL-2Rβ chain for instance the extracellular portion beginning at, or about amino acid (a,a) 1 e.g. 1, 2, 3, 4, 5, 6, 7, 8, 9, 10 and ending at or about a.a. 214 e.g. 200, 201, 202, 203, 204, 205, 206, 207, 208, 209, 210, 211, 212, 214, 215, 216, 217, 218, 219, 220, or sub-portions of this extracellular part, or portions corresponding to the intracellular part of the receptor chain e.g. the portion beginning at or about a.a. 239 e.g. a.a. 230, 231, 232, 234, 235, 236, 237, 238, 239, 240, 241, 242, up to or about the end a.a. 525, e.g. 516, 517, 518, 519, 520, 521, 522, 523, 524 and 535.

Using standard techniques of recombinant DNA technology vectors for transforming suitable host cells can be constructed which contain cDNA sequences corrsponding to the structural gene for IL-2Rβ as set forth above or any desired portion thereof, or a degenerate variant thereof.

Suitable vectors are plasmid vectors for example and will inclode control and regulatory sequences operably linked to the cDNA sequence coding for the IL-2Rβ chain or portion thereof.

Suitable techniques are well known and widely practised and by way of Example are described, in connection with other proteins in European Patent Applications, Publication Nos. 0254249 and 0170204.

Obtaining the desired portion in pure form from the culture can be carried out by standard techniques and such protein provides a suitable antigen for preparing monoclonal antibodies. Thus hybridomas capable of secreting a monoclonal antibody having a specific affinity to the IL-2Rβ chain or a desired portion thereof may be prepared by immunizing a non-human animal with recombinant IL-2Rβ or a portion thereof, removing spleen cells with non-immunoglobulin secreting myclonas cells, and selecting from the resulting hybridomas a cell line which produces a monoclonal antibody having the desired binding specificity and, if desired, subsequently sub-cloning said hybridoma.

The techniques for preparing hybridomas and obtaining monoclonal antibodies in pure form therefrom are well known and by way of example are described in European Patent Application, Publication No. 0168745.

Antibodies in accordance with the invention are useful e.g. for diagnostic purposes and also for therapy by immune suppression or activation. As mentioned above, such antibodies could be raised using purified recombinant protein in accordance with the invention or by transfecting the cDNA of the invention, obtaining cells expressing large amounts of the receptor and using such cells to obtain the antibodies.

The present invention envisages soluble forms of IL-2Rβ chain and of soluble IL-2 receptor. That is the IL-2Rβ chain may be produced in soluble form or the α-chain and β-chain produced simultaneously.

The availability of monoclonal antibodies to specific sub-portions of the IL-2β chain enables epitopes of the receptor chain to be identified and thus opens the way for control of the activity of the receptor to be excerised using suitable monoclonal antibodies or other peptides or peptide mimetic or protein analogues substances.

Expression of IL-2Rβ chain mRNA

Expression of the IL-2Rβ mRNA was examined by using the cDNA insert from pIL-2Rβ30 as the probe

Fig. 3a illustrates the expression of human IL-2Rβ chain mRNA in different cell types. Poly(A)$^+$RNA (2μg per lane) from the following cell sources was prepared and subjected to RNA blotting analysis using the XhoI-digested human IL-2Rβ chain cDNA fragment derived from pIL-2Rβ30 as a probe following standard procedures (14, 18, 27). Lane 1, YT; lane 2, Hut102(HTLV-1 transformed human T cell line); lane 3, MT-2(HTLV-1 transformed human T cell line); lane 4, ARH-77 (multible myeloma line); lane 5, SKW6.4 (EBV-tranformed human B lymphoblastoid line); lane 6, U937 (histiocytic leukemia line); lane 7, MT-1 (HTLV-1 transformed human T cell line); lane 8, Jurkat (human T leukemic line); lane 9, HeLa (human cervical carcinoma cell line.

As shown in Figure 3a, the RNA blot analysis revealed the presence of a 4kb mRNA, the expression of which is restricted to lymphoid cells previously identified to bear IL-2Rβ chain (i.e. YT, MT-2, Hut102, SKW6.4) (12, 16, 17). On the other hand, the mRNA expression was not detected in cells such as Jurkat, MT-1, U937, ARH-77 and HeLa cells. Essentially, the mRNA expression levels are in correlation with the IL-2Rβ chain expression levels.

Fig. 3 b illustrates the expression of IL-2Rβ and Il-2Rα mRNAs in human PBLs. Total RNA (15μg per lane) was loaded in each lane. Lanes 1 and 4 represents unstimulated human peripheral blood lymphocytes (PBLs); lanes 2 and 5, PBLs stimulated with 5μg/ml phytohemagglutinin (PHA) for 24 hrs; lanes 3 and 6, PBLs stimulated with 5μg/ml PHA for 72 hrs. The RNA-blotted filter was hybridized with the IL-2Rβ probe (lanes 1-3). After dehybridization of the IL-2Rβ probe, the same filter was hybridized with the IL-2Rα probe (XbaI-BclI fragment derived from pSVIL2R-3 (14) (lanes 4-6).

Interestingly, the IL-2Rβ mRNA was detectable in the unstimulated PBLs and its expression levels increased transiently only 2.5-fold after mitogen stimulation. Based on previous data derived from flow cytometric analysis (19), it is likely that the mRNA induction patterns differ between the different lymphocyte populations. This expression pattern is quite different from that of the IL-2Rα chain whose expression strictly requires mitogenic stimulation of the cells (Fig. 3b), suggesting the presence of distinct mechanisms of gene expression between the two genes.

Southern blot analysis of the genomic DNA from PBL and various cell lines including HTLV-1-transformed human T cell lines indicates that the gene is present in a single copy and is not rearranged in those cells.

IL-2 binding properties of the cDNA-encoded IL-2Rβ chain

We next carried out a series of cDNA expression studies in order to examine if the cDNA product binds IL-2 and indeed manifests the properties of the IL-2Rβ chain that have been demonstrated and/or suggested in previous studies. Two cDNA expression plasmids were constructed in which expression of the cDNA spanning the entire coding region was directed by either the mouse lck gene (29) promoter (pLCKRβ) or Moloney leukemia virus LTR (30) (pMLVRβ).

Expression vectors were constructed by the following procedures. pIL-2Rβ30 was digested with HindIII (the cleavage site is located within the polylinker regions of CDM8) and, after fill-in of both ends, a BamHI linker was attached and religated. The resulting plasmid was then digested with BamHI and the 1.8kb DNA fragment which contains the entire coding sequence for the β chain was introduced into BamHI-cleaved p1013 vector containing the mouse lck promoter to construct pLCKRβ. The BamHI-digested cDNA fragment was also introduced into a retrovirus vector, pZipSV(X) (30), to construct pMLVRβ. The human IL-2Rα expressing vector, pSVIL2Rneo, was obtained from pSVIL2R-3 (14) by replacing the Eco-gypt gene with the neo-resistance gene.

The plasmid pLCKRβ was introduced into the mouse T lymphoma EL-4 and the human T cell leukemia Jurkat lines, both of which are known to be devoid of surface molecules that bind human IL-2.

Transfection of the expression plasmids into Jurkat and EL-4 cells was carried out by electroporation as described previously (39). Transfected cells were selected in the RPMI1640 medium containing 10% fetal calf serum (FCS) and G418 (1 mg/ml for EL-4 and 1.5 mg/ml for Jurkat). To obtain cells expressing cDNAs for human IL-2Rα and IL-2Rβ chains simultaneously, a Jurkat-derived clone Jα-5, transfected with pSVIL2Rneo, was co-transfected with pLCKRB and a plasmid containing the hygromycin-resistance gene, pHgy. The transfected cells were selected with 200μg/ml hygromycin. Transfection of pMLVRβ into 2 cells was carried out by calcium-phosphate method as described previously (14) and the cells were selected by 700μg/ml of G418. For flow cytometric analysis, $5x10^5$ cells were treated with antibody (1:500 dilution of ascites) at 4°C for 30 min. After washing, cells were stained with fluorescein-conjugated goat anti-mouse IgG.

The stained cells were analysed on a FACS440 flow cytometer (Beckton Dickinson). The $^{125}$I-IL-2 binding assay and Scatchard plot analysis were carried out as described previously (12).

Fig. 4a illustrates the expression of human IL-2Rα and/or IL-2Rβ chain cDNAs by means of cell surface staining patterns of human IL-2Rα and/or IL-2Rβ cDNA transfectants. Parental cells and various transfectant cells were separately stained with either a monoclonal anti-human IL-2Rα antibody, anti-Tac (-----), or monoclonal anti-human IL-2Rβ antibody, Mik-B1 (————), Dotted line (.....) is a fluorescence profile of the cells stained with fluorescein-conjgated goat-anti-mouse IgG alone. Cells used were (1) ELB-13 (and EL-4-derived clone transfected with pLCKRβ), (2) Jβ-8 (a Jurkat-derived clone transfected with pLCKRβ), (3) Jα-5 (a Jurkat-derived clone transfected with pSVIL2Rneo), (4) Jα-2 (a Jα-5-derived clone transfected with pLCKRβ), (5) Jαβ-10 (a Jα-5-derived clone transfected with pLCKRβ), and (6) FB-3 (a NIH3T3-derived line transfected with pMLVRβ).

Stable transformant clones expressing the cDNA product were obtained for both the EL-4 (ELβ-13) and Jurkat (Jβ-8 and Jβ-9) cells as judged by FACS analysis (Fig. 4a). In addition, we also introduced the same gene into the Jurkat transformant clone, Jα-5, which expresses the transfected, human IL-2Rα chain cDNA. Two of the resulting transformants, Jαβ-2 and Jαβ-10, were found to express both α and β chains (Fig. 4a-(4), (5)). As expected, RNA blotting analyses of the mRNA expressed in those transformants revealed that the α and β chain-specific mRNAs are derived from the transfected cDNAs but not from the endogeneous genes (26). Furthermore, in order to examine the property of the cDNA product in non-lymphoid cells, the plasmid pMLVRβ was introduced into an NIH3T3 cell-derived cell line 2 (30), and the resulting transformant expressing the cDNA, Fβ-3, was obtained (Fig. 4a-(5)).

The IL-2 binding studies were carried out with $^{125}$I-labeled, recombinant human IL-2.

Fig. 4b illustrates the expression of the α and β chains by means of the Scatchard plot analysis of $^{125}$I-IL-2 binding to the transfectants expressing the cloned cDNAs. Scatchard plot of the IL-2 binding data in the absence ( o—o ) or presence ( •—• ) of 1:100-diluted ascites of Mik-β1. Binding of $^{125}$I-IL-2 to ELβ-13 or Jβ-8 was completely abolished by Mik-β1. No specific IL-2 binding was observed when parental Jurkat or EL-4 cells were examined. The number of IL-2 binding sites per cell and the receptor affinity were determined by computer-assisted analysis of the IL-2 binding data. (1) ELβ-13, (2) Jβ-8, (3) Jα-5, (4) Jαβ-2, (5) Jαβ-10.

As can be seen the EL-4-derived clone (ELβ-13) and the Jurkat-derived clone (Jβ-8), both expressing the β chain cDNA displayed intermediate-affinity to IL-2 with estimated Kd values of 4.0nM and 2.7nM, respectively. The IL-2 binding to those cells was completely abolished by the Mik-β1 antibody (Fig. 4b-(1), (2)). The Jurkat-derived Jαβ-2 and Jαβ-10 clones expressing both the human IL-2Rα and Il-2Rβ cDNA displayed both high and low affinity receptors with estimated Kp values of 22pM and l5nM for Jαβ-2 and 19pM and 33nM for Jαβ-10, respectively. In contrast, the parental, Jurkat-derived Jα-5 cells expressing the α chain cDNA alone manifested exclusively low-affinity (Kd: 19.5nM) to IL-2 (Fig. 4b-(3)). the number of the high-affinity IL-2R expressed Jαβ-2 cells and Jαβ-10 was comparable to that of expressed IL-2Rβ molecules. In addition, treatment of these cells with Mik-β1 antibody completely abolished high-affinity IL-2 binding sites from the cell surface, while retaining the expression of low-affinity IL-2R (Fig. 4b-(4), (5)). These observations demonstrate unequivocally that the cDNA-encoded IL-2Rβ molecule is directly involved in the formation of high-affinity receptor complex in association with the IL-2Rα chain. In contrast to the aforedescribed T cell transformants, the Fβ-3 cells did not display any IL-2 binding on the cell surface under same binding conditions. Interestingly the same observation was made with monkey COS cells that express the β chain, but failed to bind IL-2 (28). Thus, the results suggest the involvement of either a cell-type specific processing mechanism(s) or an additional cellular component(s), or both for the functional IL-2Rβ chain expression.

In order to characterize further the molecular structure of reconstituted IL-2R, we performed chemical crosslinking experiments with $^{125}$I-IL-2 and non-cleavable chemical crosslinker, dissuccinimidyl suberate (DSS).

Fig. 5 illustrates the results of the affinity cross-linking studies of the IL-2R-positive transformants. Cells were incubated with 5nM (lanes 1-13) or 100pM (lanes 14-16) of $^{125}$I-IL-2 in the absence (lanes 1-4, 14-16) or presence of a 250-fold molar excess of unlabeled IL-2 (lanes 5-7), 500-fold molar excess of affinity column-purified Mik-β1 (lanes 8-10) or 500-fold molar excess of affinity column-purified anti-Tac (lanes 11-13). Then cells were chemically crosslinked with dissuccinimidyl suberate (DSS) as described previously (16). The cells were then solubilized and the supernatants were subjected to 7.5% SDS-PAGE. Cells used were: Jurkat (lane 1); Jα-5 (lanes 2, 5, 8, 11, 14); Jβ-8 (lanes 3, 6, 9, 12, 15); Jαβ-10 (lanes 4, 7, 10, 13, 16). YT cells crosslinked with $^{125}$I-IL-2 were used as a marker (M).

As can be seen cells expressing only IL-2Rβ chain were crosslinked with $^{125}$I-labeled IL-2 and analysed by SDS-PAGE, a doublet band consisting of 90kD major and 85kD minor was detected and its migration profile was indistinguishable from that of YT cells (see arrows in Fig. 5 and ref. 16, 17). The appearance of the doublet is inhibited by an excess of unlabeled IL-2 or by Mik-β1. The doublet formation may be due to degradation of receptor-IL-2 complex. It is also possible that both protein products are derived by a differential post-translational modification(s). Alternatively, one of the doublet may represent a third component of the receptor complex. A broad band migrating around the position of 150kD was also detected in the transfectant (Jαβ-10) as well as YT cells. The appearance of this band is also inhibited by either unlabeled IL-2 or Mik-β1. It may represent the ternary complex of IL-2, IL-2Rα and Il-2Rβ molecules. In a series of chemical cross-linking experiments shown in Fig. 4, it was demonstrated that the physico-chemical properties of the receptor complex expressed on the surface of Jαβ-2 are indistinguishable from the properties of high-affinity receptor expressed on cultured T cells or PBLs (12, 16, 17).

Preliminary results of experiments to determine whether the expression of the α and β chains in non-lymphoid cells results in the formation of high-affinity receptor indicate that, when the α and β chain cDNAs are co-expressed transiently in COS cells, both chains can crosslink with $^{125}$I-IL-2 at the concentration (400 pM) in which the similarly expressed α chain alone can not (28). The results may suggest the formation of the αβ heterodimeric receptor in this non-lymphoid cell line.

IL-2 internalization by reconstituted receptors

It has been reported that intermediate- and high-affinity IL-2 receptors can both internalize IL-2 (33-35). Ligand internalization is usually accompanied with the IL-2 signal transduction, suggesting this process to be essential.

Fig. 6 illustrates IL-2 internalization via the reconstituted receptors. IL-2 internalization was examined according to a method described previously (33). Briefly, cells (5x10$^7$) were treated with $^{125}$I-IL-2 at a final concentration of 200pM (Jαβ-10) or 5nM (Jα-5, Jβ-8 and ELβ-13) at 0°C for 30 min. After washing, cells were suspended with prewarmed culture medium (37°C) and the kinetics of IL-2 internalization was examined as described previously (33). (a) ELβ-13, (b) Jβ-8, (c) Jαβ-10, (d) Jα-5. (-●-●-●-), internalized IL-2; (...O...O...), cell-surface bound IL-2; (-■- – -■-), free IL-2.

As shown in Fig. 6, we examined whether the reconstituted receptors can internalize IL-2. In fact, the cells expressing IL-2Rβ chain alone, or both α and β chains are capable of internalizing IL-2 following a kinetic pattern similar to that reported for the native receptor. In contrast, the Jurkat cells expressing only IL-2Rα failed to internalize IL-2, similar to previously reported observations (33, 34). Preliminary results indicate that the growth of the cells expressing the intermediate- or high-affinity receptors is selectively inhibited by IL-2 (14, 36). We also have preliminary results that the β chain expressed in another host cell line functions in stimulating the cell growth in response to IL-2 (28).

The availability of the gene encoding the IL-2Rβ chain makes it possible to explore novel approaches for the functional studies of the IL-2 system. The receptor structure operating in the IL-2 system is unique in that two structurally distinct membrane molecules, the IL-2Rα and IL-2Rβ chains, both bind IL-2 independently. The series of cDNA expression examples described herein substantiate further the previous notion that the α and β chains constitute the high-affinity IL-2R complex via a non-covalent association of the molecules (18, 37). Thus the peculiarity of this system is the involvement of three intermolecular interactions between one ligand and two distinct receptors. By virtue of the present invention it will now be possible to elucidate functional domains of this unique cytokine receptor system. Mutational analyses of the cloned β chain cDNA may provide clues as to the identification of respective domains involved in ligand binding and association with the α chain. To date, little is known about the cascade of biochemical events triggered by cytokines interacting with their homologous receptors. By the present invention we have demonstrate the presence in the IL-2Rβ chain of a large cytoplasmic region which most likely is involved in driving the IL-2 signal pathway(s). The particular acidic nuclei found in the cytoplasmic region may suggest coupling to other cytoplasmic signal transducers. Alternatively, in view of a previous report on the presence of IL-2 within the nucleous (33), an intriguing possibility is that the acidic as well as the proline-rich regions of the IL-2R$\beta$ cytoplasmic component may play a role in activation of the genetic programming. The availability of the expression system in which the cDNA-encoded $\beta$ chain can deliver growth signals will allow further clarification of the functional domaines of the receptor. It is now possible to study the essential role of IL-2 in the development and regulation of the immune system.

REFERENCES AND NOTES

1. D.A. Morgan, F.W. Ruscelli, R.C. Gallo, Science 198, 1007 (1976)
2.K.A. Smith, Annu.Rev.Immunol. 2, 319 (1984); T. Taniguchi et al., Immunol.Rev. 92, 121 (1986)
3. D.H. Raulet, Nature 314, 101 (1985)
4. M. Tsudo, T. Uchiyama, H. Uchino, J.Exp.Med. 160, 612 (1984); T.A. Waldmann et al., ibid., p. 1450 (1984); M.A. Blackman, M.A. Tigges, M.E. Minis, M.E. Koshland, Cell, 47, 609 (1986)
5. M. Malkovsky et al., Nature 325, 262 (1987)
6. C.S. Henney, K. Kuribayashi, D.E. Kern, S. Gillis, ibid. 291, 335 (1981)
7. M.E. Lotze, E.A. Grimm, S.A. Strausser, S.A. Rosenberg, Cancer Res. 41, 4420 (1981); E.A. Grimm, A. Mazumder, H.Z. Zhang, S.A. Rosenberg, J.EXP. Med. 155, 1823 (1982
8. S.A. Rosenberg et al., N.Engl.J.Med. 316, 889 (1987)
9. E.N. Benveniste, J.E. Merrill, Nature 321, 610 (1986)
10. S.J. LeGrue, Lymphokine Res. 7, 1987 (1988); G.B. Millis, P. Girard, S. Grinstein, E.W. Gelfand, Cell 55, 91 (1988); V.E. Valge, J.G.P. Wong, B.M. Datlof, A.J. Sinskey, A. Rao, ibid., p. 101 (1988); M.A. Tigges, L.S. Casey, M.E. Koshland, Science 243, 781 (1989)
11. R.J. Robb, W.C. Greene, C.M. Rusk, J.Exp.Med. 160, 1126 (1984)
12. M. Tsudo, R.W. Kozak, C.K. Goldman, T.A. Waldmann, Proc.Natl.Acad.Sci.U.S.A. 83, 9694 (1986); K. Teshigawara, H.-M. Wang, K.Kato, K.A. Smith, J.Exp.Med. 165, 223 (1987)
13. W.J. Leonard et al., Nature 311, 626 (1984) T. Nikaido et al., ibid., p. 631 (1984); D. Cosman et al., ibid. 312, 768 (1984)
14. M. Hatakeyama et al., ibid. 318, 467 (1985)
15. W.C. Greene et al., J. Exp. Med. 162, 363 (1985), S. Kondo et al., ibid. 320, 75 (1986); R.J. Robb, Proc.Natl. Acad. Sci.U.S.A. 83, 3992 (1986)
16. M. Sharon, R.D. Klausner, B.R. Cullen, R. Chizzonite, W.J. Leonard, Science 234, 859 (1986)
17. R.J. Robb et al., Proc.Natl.Acad.Sci.U.S.A., 84, 2002 (1987); M. Tsudo, R.W. Kozak, C.K. Goldman, T.A. Waldmann, ibid., p 4215 (1987); M. Dukovich et al., Nature 327, 518 (1987)
18. M. Hatakeyama et al., J.Exp.Med. 166, 362 (1987); S. Kondo et al., Nature 327, 64 (1987)
19. M. Tsudo, F. Kitamura, M. Miyasaka, Proc.Natl.Acad.Sci.U.S.A. in press.
20. J. Yodoi et al., J.Immunol. 134, 1623 (1985)
21. B. Seed, A. Aruffo, Proc.Natl.Acad.Sci.U.S.A. 84, 3365 (1987); B. Seed, Nature 328, 840 (1987)
22. D. Perlman, H.O. Halvorson, J.Mol.Biol. 167, 391 (1983); G. von Heijne, Nucleic Acids Res. 14, 4683 (1986)
23. A. Ullrich et al., Nature 309, 418 (1984); A. Ullrich et al., ibid. 313, 756 (1985); U. Ebina et al., Cell 40, 747 (1985)
24. L. Collins et al., Proc.Natl.Acad.Sci.U.S.A. 85, 7709 (1988)
25. K.S. Hanks, A.M. Quinn, T. Hunter, Science 241, 42 (1988)
26. A. Alcover et al., Immunol.Rev. 95,5 (1987)
27. M. Hatakeyama, S. Minamoto, T. Taniguchi, Proc.Natl.Acad.Sci.U.S.A. 83, 9650 (1986)
28. M. Hatakeyama, T. Doi, T. Kono, S. Minamoto, T. Taniguchi, unpublished observations
29. J.D. Marth, R. Peet, E.G. Krebs, R.M. Perlmutter, Cell 43, 393 (1985)
30. R. Mann, R.C. Mulligan, D. Baltimore, ibid. 33, 153 (1983)
31. M. Fujii et al., J.Exp.Med. 163, 550 (1986)
32. A.M Weissman et al., Proc.Natl.Acad.Sci.U.S.A. 83, 1463 (1986)
33. R.J Robb, W.C. Greene, J.Exp.Med. 165, 1201 (1987)
34. K Sugamura et 1., ibid. 161, 1243 (1985)
35. H Saragovi, T.R. Malek, J.Immunol. 141, 476 (1988)
36. J Kyte, R.F. Doolittle, J.Mol.Biol. 157, 105 (1982)
37. H Potter, L. Weir, P. Leder, Proc.Natl.Acad.Sci.U.S.A. 81, 7161 (1984)

## Claims

1. A recombinant DNA molecule coding for the β-chain of the IL-2 receptor or a portion thereof.
2. A recombinant DNA molecule characterized by a structural gene having the formula:

ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG
AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG
ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC

TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT
GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG
GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA
ATC CAC TTG GTG TAG

or a portion thereof or a degenerate variant thereof.

3. A recombinant DNA molecule according to claim 2 characterized by a DNA sequence having the formula:

GCAGCCAGAGCTCAGCAGGGCCCTGGAGAGATGG
CCACGGTCCCAGCACCGGGGAGGACTGGAGAGCGCGCGCTGCCACCGCCCC
ATGTCTCAGCCAGGGCTTCCTTCCTCGGCTCCACCCTGTGGATGTA ATG
GCG GCC CCT GCT CTG TCC TGG CGT CTG CCC CTC CTC ATC
CTC CTC CTG CCC CTG GCT ACC TCT TGG GCA TCT GCA GCG
GTG AAT GGC ACT TCC CAG TTC AGA TGC TTC TAC AAC TCG
AGA GCC AAC ATC TCC TGT CTC TGG AGC CAA GAT GGG GCT
CTG CAG GAC ACT TCC TGC CAA GTC CAT GCC TGG CCG GAC
AGA CGG CGG TGG AAC CAA ACC TGT GAG CTG CTC CCC GTG
AGT CAA GCA TCC TGG GCC TGC AAC CTG ATC CTC GGA GCC
CCA GAT TCT CAG AAA CTG ACC ACA GTT GAC ATC GTC ACC
CTG AGG GTG CTG TGC CGT GAG GGG GTG CGA TGG AGG GTG
ATG GCC ATC CAG GAC TTC AAG CCC TTT GAG AAC CTT CGC
CTG ATG GCC CCC ATC TCC CTC CAA GTT GTC CAC GTG GAG
ACC CAC AGA TGG AAC ATA AGC TGG GAA ATC TCC CAA GCC
TCC CAC TAC TTT GAA AGA CAC CTG GAG TTC GAG GCC CGG

ACG CTG TCC CCA GGC CAC ACC TGG GAG GAG GCC CCC CTG
CTG ACT CTC AAG CAG AAG CAG GAA TGG ATC TGC CTG GAG
ACG CTC ACC CCA GAC ACC CAG TAT GAG TTT CAG GTG CGG
GTC AAG CCT CTG CAA GGC GAG TTC ACG ACC TGG AGC CCC
TGG AGC CAG CCC CTG GCC TTC AGG ACA AAG CCT GCA GCC
CTT GGG AAG GAC ACC ATT CCG TGG CTC GGC CAC CTC CTC
GTG GGC CTC AGC GGG GCT TTT GGC TTC ATC ATC TTA GTG
TAC TTG CTG ATC AAC TGC AGG AAC ACC GGG CCA TGG CTG
AAG AAG CTC CTG AAG TGT AAC ACC CCA GAC CCC TCG AAG
TTC TTT TCC CAG CTG AGC TCA GAG CAT GGA GGA GAC GTC
CAG AAG TGG CTC TCT TCG CCC TTC CCC TGA TCG TCC TTC
AGC CCT GGC GGC CTG GCA CCT GAG ATC TCG CCA CTA GAA
GTG CTG GAG AGG GAC AAG GTG ACG CAG CTG CTC CTG CAG
CAG GAC AAG GTG CCT GAG CCC GCA TCC TTA AGC AGC AAC
CAC TCG CTG ACC AGC TGC TTC ACC AAC CAG GGT TAC TTC
TTC TTC CAC CTC CCG GAT GCC TTG GAG ATA GAG GCC TGC
CAG GTG TAC TTT ACT TAC GAC CCC TAC TCA GAG GAA GAC
CCT GAT GAG GGT GTG GCC GGG GCA CCC ACA GGG TCT TCC
CCC CAA CCC CTG CAG CCT CTG TCA GGG GAG GAC GAC GCC
TAC TGC ACC TTC CCC TCC AGG GAT GAC CTG CTG CTC TTC
TCC CCC AGT CTC CTC GGT GGC CCC AGC CCC CCA AGC ACT
GCC CCT GGG GGC AGT GGG GCC GGT GAA GAG AGG ATG CCC
CCT TCT TTG CAA GAA AGA GTC CCC AGA GAC TGG GAC CCC
CAG CCC CTG GGG CCT CCC ACC CCA GGA GTC CCA GAC CTG
GTG GAT TTT CAG CCA CCC CCT GAG CTG GTG CTG CGA GAG
GCT GGG GAG GAG GTC CCT GAC GCT GGC CCC AGG GAG GGA
GTC AGT TTC CCC TGG TCC AGG CCT CCT GGG CAG GGG GAG
TTC AGG GCC CTT AAT GCT CGC CTG CCC CTG AAC ACT GAT
GCC TAC TTG TCC CTC CAA GAA CTC CAG GGT CAG GAC CCA
ATC CAC TTG GTG TAG ACAGATGGCCAGGGTGGGAGGCAGGCAGCT
GCCTGCTCTGCGCCGAGCCTCAGAAGGACCCTGTTGAGGGTCCTCAGTCCA
CTGCTGAGGACACTCAGTGTCCAGTTGCAGCTGGACTTCTCCACCCGGATG
GCCCCCACCCAGTCCTGCACACTTGGTCCATCCATTTCCAAACCTCCACTG
CTGCTCCCGGGTCCTGCTGCCCGAGCCAGGAACTGTGTGTGTTGCAGGGGG
GCAGTAACTCCCCAACTCCCTCGTTAATCACAGGATCCCACGAATTTAGGC
TCAGAAGCATCGCTCCTCTCCAGCCCTGCAGCTATTCACCAATATCAGTCC
TCGCGGCTCTCCAGGGCTCCCTGCCCTGACCTCTTCCCTGGGTTTTCTGCC

CCAGCCTCCTCCTTCCCTCCCCTCCCCGTCCACAGGGCAGCCTGAGCGTGC
TTTCCAAAACCCAAATATGGCCACGCTCCCCCTCGGTTCAAAACCTTGCAC
AGGTCCCACTGCCCTCAGCCCCACTTCTCAGCCTGGTACTTGTACCTCCGG
TGTCGTGTGGGACATCCCCTTCTGCAATCCTCCCTACCGTCCTCCCGAGC
CACTCAGAGCTCCCTCACACCCCCTCTGTTGCACATGCTATTCCCTGGGGC
TGCTGTGCGCTCCCCCTCATCTAGGTGACAAACTTCCCTGACTCTTCAAGT
GCCGGTTTTGCTTCTCCTGGAGGGAAGCACTGCCTCCCTTAATCTGCCAGA
AACTTCTAGCGTCAGTGCTGGAGGGAGAAGCTGTCAGGGACCCAGGGCGCC
TGGAGAAAGAGGCCCTGTTACTATTCCTTTGGGATCTCTGAGGCCTCAGAG
TGCTTGGCTGCTGTATCTTTAATGCTGGGGCCCAAGTAAGGGCACAGATCC
CCCCGACAAAGTGGATGCCTGCTGCATCTTCCCACAGTGGCTTCACAGACC
CACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACT
GCCCCCTCCACTGGAGGGGTCTCCTCTGCAGCTCAGGGACTGGCACACTGG
CCTCCAGAAGGGCAGCTCCACAGGGCAGGGCCTCATTATTTTCACTGCCC
CAGACACAGTGCCCAACACCCCGTCGTATACCCTGGATGAACGAATTAATT
ACCTGGCACCACCTCGTCTGGGCTCCCTGCGCCTGACATTCACACAGAGAG
GCAGAGTCCCGTGCCCATTAGGTCTGGCATGCCCCCTCCTGCAAGGGGCTC
AACCCCCTACCCCGACCCCTCCACGTATCTTTCCTAGGCAGATCACGTTGC
AATGGCTCAAACAACATTCCACCCCAGCAGGACAGTGACCCCAGTCCCAGC
TAACTCTGACCTGGGAGCCCTCAGGCACCTGCACTTACAGGCCTTGCTCAC
AGCTGATTGGGCACCTGACCACACGCCCCCACAGGCTCTGACCAGCAGCCT
ATGAGGGGGTTTGGCACCAAGCTCTGTCCAATCAGGTAGGCTGGGCCTGAA
CTAGCCAATCAGATCAACTCTGTCTTGGGCGTTTGAACTCAGGGAGGGAGG
CCCTTGGGAGCAGGTGCTTGTGGACAAGGCTCCACAAGCGTTGAGCCTTGG
AAAGGTAGACAAGCGTTGAGCCACTAAGCAGAGGACCTTGGGTTCCCAATA
CAAAAATACCTACTGCTGAGAGGGCTGCTGACCATTTGGTCAGGATTCCTG
TTGCCTTTATATCCAAAATAAACTCCCCTTTCTTGAGGTTGTCTGAGTCTT
GGGTCTATGCCTTGAAAAAAGCTGAATTATTGGACAGTCTCACCTCCTGCC
ATAGGGTCCTGAATGTTTCAGACCACAAGGGGCTCCACACCTTTGCTGTGT
GTTCTGGGGCAACCTACTAATCCTCTCTGCAAGTCGGTCTCCTTATCCCCC
CAAATGGAAATTGTATTTGCCTTCTCCACTTTGGGAGGCTCCCACTTCTTG
GGAGGGTTACATTTTTTAAGTCTTAATCATTTGTGACATATGTATCTATAC
ATCCGTATCTTTTAATGATCCGTGTGTACCATCTTTGTGATTATTTCCTTA
ATATTTTTTCTTTAAGTCAGTTCATTTTCGTTGAAATACATTTATAAAGAA
AAATCTTTGTTACTCTGTAAATGAAAAAACCCATTTTCGCTATAAATAAAA
GGTAACTGTACAAAATAAGTACAAT

or a portion thereof or a degenerate variant thereof.

4. A recombinant DNA molecule as defined in any one of claims 1 to 3 which further comprises regulatory sequences operably linked to the structural gene for the IL-2β chain or portion thereof.

5. A recombinant DNA molecule as defined in claim 4 which is a plasmid.

6. A recombinant DNA molecule as defined in claim 5, this being one of the following
pIL-2Rβ6,
pIL-2Rβ9,
pIL-2Rβ19,
pIL-2Rβ30.

7. A host cell which has been transformed by a recombinant DNA molecule as defined in any one of claims 1 to 6.

8. A host cell as defined in claim 7, which is a bacterial cell or a yeast cell or a mammalian cell.

9 A protein having the structure defined by the structural gene set forth in claim 2 or a portion thereof.

10. A hybridoma, sub-clone or mutant thereof capable of secreting a monoclonal antibody having a specific affinity to a protein as defined in claim 9.

11. A monoclonal antibody having a specific affinity to a protein as defined in claim 9.

12. A method of producing a hybridoma as defined in claim 10 which comprises immunizing a non-human animal with a protein as defined in claim 9, removing spleen cells from the immunized animal and fusing the spleen cells with non-immunoglobulin secreting myloma cells, and selecting from the resulting hybridomas a cell line which produces a monoclonal antibody having the desired binding specificity and, if desired, subsequently sub-cloning said hybridoma.

Nouvellement déposé

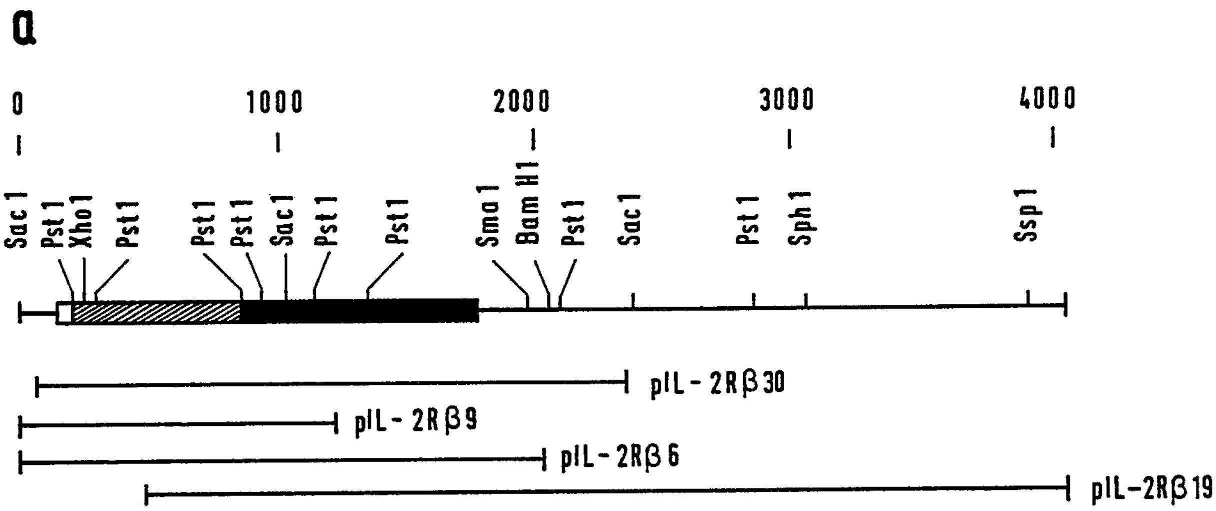


FIG. 1a

Fig. 1b

```
                    GCAGCCAGAGCTCAGCAGGGCCCTGGAGAGATGGCCA                37
CGGTCCCAGCACCGGGGAGGACTGGAGAGCGCGCGCTGCCACCGCCCCA
TGTCTCAGCCAGGGCTTCCTTCCTCGGCTCCACCCTGTGGATGTA ATG                   134
                                              Met
    GCG GCC CCT GCT CTG TCC TGG CGT CTG CCC CTC CTC ATC              173
-25 Ala Ala Pro Ala Leu Ser Trp Arg Leu Pro Leu Leu Ile
                                                -26
    CTC CTC CTG CCC CTG GCT ACC TCT TGG GCA TCT GCA GCG              212
-12 Leu Leu Leu Pro Leu Ala Thr Ser Trp Ala Ser Ala Ala
    GTG AAT GGC ACT TCC CAG TTC ACA TGC TTC TAC AAC TCG              251
  2 Val AsN Gly Thr Ser GlN Phe Thr Cys Phe Tyr AsN Ser
    AGA GCC AAC ATC TCC TGT CTC TGG AGC CAA GAT GGG GCT              290
 15 Arg Ala AsN Ile Ser Cys Val Trp Ser GlN Asp Gly Ala
    CTG CAG GAC ACT TCC TGC CAA GTC CAT GCC TGG CCG GAC              329
 28 Leu GlN Asp Thr Ser Cys GlN Val His Ala Trp Pro Asp
    AGA CGG CGG TGG AAC CAA ACC TGT GAG CTG CTC CCC GTG              368
 41 Arg Arg Arg Trp AsN GlN Thr Cys Glu Leu Leu Pro Val
    AGT CAA GCA TCC TGG GCC TGC AAC CTG ATC CTC GGA GCC              407
 54 Ser GlN Ala Ser Trp Ala Cys AsN Leu Ile Leu Gly Ala
    CCA GAT TCT CAG AAA CTG ACC ACA GTT GAC ATC GTC ACC              446
 67 Pro Asp Ser GlN Lys Leu Thr Thr Val Asp Ile Val Thr
    CTG AGG GTG CTG TGC CGT GAG GGG GTG CGA TGG AGG GTG              485
 80 Leu Arg Val Leu Cys Arg Glu Gly Val Arg Trp Arg Val
    ATG GCC ATC CAG GAC TTC AAG CCC TTT GAG AAC CTT CGC              524
 93 Met Ala Ile GlN Asp Phe Lys Pro Phe Glu AsN Leu Arg
    CTG ATG GCC CCC ATC TCC CTC CAA GTT GTC CAC GTG GAG              563
106 Leu Met Ala Pro Ile Ser Leu GlN Val Val His Val Glu
    ACC CAC AGA TGG AAC ATA AGC TGG GAA ATC TCC CAA GCC              602
119 Thr His Arg Cys AsN Ile Ser Trp Glu Ile Ser GlN Ala
    TCC CAC TAC TTT GAA AGA CAC CTG GAG TTC GAG GCC CGG              641
132 Ser His Tyr Phe Glu Arg His Leu Glu Phe Glu Ala Arg
    ACG CTG TCC CCA GGC CAC ACC TGG GAG GAG GCC CCC CTG              680
145 Thr Leu Ser Pro Gly His Thr Trp Glu Glu Ala Pro Leu
    CTG ACT CTC AAG CAG AAG CAG GAA TGG ATC TGC CTG GAG              719
158 Leu Thr Leu Lys GlN Lys GlN Glu Trp Ile Cys Leu Glu
    ACG CTC ACC CCA GAC ACC CAG TAT GAG TTT CAG GTG CGG              758
171 Thr Leu Thr Pro Asp Thr GlN Tyr Glu Phe GlN Val Arg
    GTC AAG CCT CTG CAA GGC GAG TTC ACG ACC TGG AGC CCC              797
184 Val Lys Pro Leu GlN Gly Glu Phe Thr Thr Trp Ser Pro
```

Fig. 1b cont'd

TGG AGC CAG CCC CTG GCC TTC AGG ACA AAG CCT GCA GCC 836
197 Trp Ser GlN Pro Leu Ala Phe Arg Thr Lys Pro Ala Ala

CTT GGG AAG GAC ACC ATT CCG TGG CTC GGC CAC CTC CTC 875
210 Leu Gly Lys Asp Thr Ile Pro Trp Leu Gly His Leu Leu

GTG GGC CTC AGC GGG GCT TTT GGC TTC ATC ATC TTA GTG 914
223 Val Gly Leu Ser Gly Ala Phe Gly Phe Ile Ile Leu Val

TAC TTG CTG ATC AAC TGC AGG AAC ACC GGG CCA TGG CTG 953
236 Tyr Leu Leu Ile AsN Cys Arg AsN Thr Gly Pro Trp Leu

AAG AAG CTC CTG AAG TGT AAC ACC CCA GAC CCC TCG AAG 992
249 Lys Lys Val Leu Lys Cys AsN Thr Pro Asp Pro Ser Lys

TTC TTT TCC CAG CTG AGC TCA GAG CAT GGA GGA GAC GTC 1031
262 Phe Phe Ser GlN Leu Ser Ser Glu His Gly Gly Asp Val

CAG AAG TGG CTC TCT TCG CCC TTC CCC TCA TCG TCC TTC 1070
275 GlN Lys Trp Leu Ser Ser Pro Phe Pro Ser Ser Ser Phe

AGC CCT GGC GGC CTG GCA CCT GAG ATC TCG CCA CTA GAA 1109
286 Ser Pro Gly Gly Leu Ala Pro Glu Ile Ser Pro Leu Glu

GTG CTG GAG AGG GAC AAG GTG ACG CAG CTG CTC CTG CAG 1148
301 Val Leu Glu Arg Asp Lys Val Thr GlN Leu Leu Leu GlN

CAG GAC AAG GTG CCT GAG CCC GCA TCC TTA AGC AGC AAC 1187
314 GlN Asp Lys Val Pro Glu Pro Ala Ser Leu Ser Ser AsN

CAC TCG CTG ACC AGC TGC TTC ACC AAC CAG GGT TAC TTC 1226
327 His Ser Leu Thr Ser Cys Phe Thr AsN GlN Gly Tyr Phe

TTC TTC CAC CTC CCG GAT GCC TTG GAG ATA GAG GCC TGC 1265
340 Phe Phe His Leu Pro Asp Ala Leu Glu Ile Glu Ala Cys

CAG GTG TAC TTT ACT TAC GAC CCC TAC TCA GAG GAA GAC 1304
353 GlN Val Tyr Phe Thr Tyr Asp Pro Tyr Ser Glu Glu Asp

CCT GAT GAG GGT GTG GCC GGG GCA CCC ACA GGG TCT TCC 1343
366 Pro Asp Glu Gly Val Ala Gly Ala Pro Thr Gly Ser Ser

CCC CAA CCC CTG CAG CCT CTG TCA GGG GAG GAC GAC GCC 1382
379 Pro GlN Pro Leu GlN Pro Leu Ser Gly Glu Asp Asp Ala

TAC TGC ACC TTC CCC TCC AGG GAT GAC CTG CTG CTC TTC 1421
392 Tyr Cys Thr Phe Pro Ser Arg Asp Asp Leu Leu Leu Phe

TCC CCC AGT CTC CTC GGT GGC CCC AGC CCC CCA AGC ACT 1460
405 Ser Pro Ser Leu Leu Gly Gly Pro Ser Pro Pro Ser Thr

Fig. 1b cont'd

| | | |
|---|---|---|
| | GCC CCT GGG GGC AGT GGG GCC GGT GAA GAG AGG ATG CCC | 1499 |
| 418 | Ala Pro Gly Gly Ser Gly Ala Gly Glu Glu Arg Met Pro | |
| | CCT TCT TTG CAA GAA AGA GTC CCC AGA GAC TGG GAC CCC | 1538 |
| 431 | Pro Ser Leu GlN Glu Arg Val Pro Arg Asp Trp Asp Pro | |
| | CAG CCC CTG GGG CCT CCC ACC CCA GGA GTC CCA GAC CTG | 1577 |
| 444 | GlN Pro Leu Gly Pro Pro Thr Pro Gly Val Pro Asp Leu | |
| | GTG GAT TTT CAG CCA CCC CCT GAG CTG GTG CTG CGA GAG | 1616 |
| 457 | Val Asp Phe GlN Pro Pro Pro Glu Leu Val Leu Arg Glu | |
| | GCT GGG GAG GAG GTC CCT GAC GCT GGC CCC AGG GAG GGA | 1655 |
| 470 | Ala Gly Glu Glu Val Pro Asp Ala Gly Pro Arg Glu Gly | |
| | GTC AGT TTC CCC TGG TCC AGG CCT CCT GGG CAG GGG GAG | 1694 |
| 483 | Val Ser Phe Pro Trp Ser Arg Pro Pro Gly GlN Gly Glu | |
| | TTC AGG GCC CTT AAT GCT CGC CTG CCC CTG AAC ACT GAT | 1733 |
| 496 | Phe Arg Ala Leu AsN Ala Arg Leu Pro Leu AsN Thr Asp | |
| | GCC TAC TTG TCC CTC CAA GAA CTC CAG GGT CAG GAC CCA | 1772 |
| 509 | Ala Tyr Leu Ser Leu GlN Glu Leu GlN Gly GlN Asp Pro | |
| | ACT CAC TTG GTG TAG ACAGATGGCCAGGGTGGAGGCAGGCAGCT | 1817 |
| 522 | Thr His Leu Val *** | |
| | GCCTGCTCTGCGCCGAGCCTCAGAAGGACCCTGTTGAGGGTCCTCAGTCCA | 1868 |
| | CTGCTGAGGACAC TCAGTGTCCAGTTGCAGCTGGACTTCTCCACCCGGAT | 1918 |
| | GGCCCCCACCCAGTCCTGCACACTTGGTCCATCCATTTCCAAACCTCCACT | 1969 |
| | GCTGCTCCCGGGTCCTGCTGCCCGAGCCAGGAACTGTGTGTGTTGCAGGGG | 2020 |
| | GGCAGTAACTCCCCAACTCCCTCGTTAATCACAGGATCCCACGAATTTAGG | 2071 |
| | CTCAGAAGCATCGCTCCTCTCCAGCCCTGCAGCTATTCACCAATATCAGTC | 2122 |
| | CTCGCGGCTCTCCAGGGCTCCCTGCCCTGACCTCTTCCCTGGGTTTTCTGC | 2173 |
| | CCCAGCCTCCTCCTTCCCTCCCCTCCCCGTCCACAGGGCAGCCTGAGCGTG | 2224 |
| | CTTTCCAAAACCCAAATATGGCCACGCTCCCCCTCGGTTCAAAACCTTGCA | 2275 |
| | CAGGTCCCACTGCCCTCAGCCCCACTTCTCAGCCTGGTACTTGTACCTCCG | 2326 |
| | GTGTCGTGTGGGGACATCCCCTTCTGCAATCCTCCCTACCGTCCTCCCGAG | 2377 |
| | CCACTCAGAGCTCCCTCACACCCCCTCTGTTGCACATGCTATTCCCTGGGG | 2428 |

Fig. 1b cont'd

CTGCTGTGCGCTCCCCCTCATCTAGGTGACAAACTTCCCTGACTCTTCAAG 2479
TGCCGGTTTTGCTTCTCCTGGAGGGAAGCACTGCCTCCCTTAATCTGCCAG 2530
AAACTTCTAGCGTCAGTGCTGGAGGGAGAAGCTGTCAGGGACCCAGGGCGC 2581
CTGGAGAAAGAGGCCCTGTTACTATTCCTTTGGGATCTCTGAGGCCTCAGA 2632
GTGCTTGGCTGCTGTATCTTTAATGCTGGGGCCCAAGTAAGGGCACAGATC 2683
CCCCCACAAAGTGGATGCCTGCTGCATCTTCCCACAGTGGCTTCACAGACC 2734
CACAAGAGAAGCTGATGGGGAGTAAACCCTGGAGTCCGAGGCCCAGGCAGC 2785
AGCCCCGCCTAGTGGTGGGCCCTGATGCTGCCAGGCCTGGGACCTCCCACT 2836
GCCCCCTCCACTGGAGGGGTCTCCTCTGCAGCTCAGGGACTGGCACACTGG 2887
CCTCCAGAAGGGCAGCTCCACAGGGCAGGGCCTCATTATTTTCACTGCCC 2938
CAGACACAGTGCCCAACACCCCGTCGTATACCCTGGATGAACGAATTAATT 2989
ACCTGGCACCACCTCGTCTGGGCTCCCTGCGCCTGACATTCACACAGAGAG 3040
GCAGAGTCCCGTGCCCATTAGGTCTGGCATGCCCCCTCCTGCAAGGGGCTCA 3092
ACCCCCTACCCCGACCCCTCCACGTATCTTTCCTAGGCAGATCACGTTGCAA 3144
TGGCTCAAACAACATTCCACCCCAGCAGGACAGTGACCCCAGTCCCAGCTAA 3196
CTCTGACCTGGGAGCCCTCAGGCACCTGCACTTACAGGCCTTGCTCACAGCT 3248
GATTGGGCACCTGACCACACGCCCCCACAGGCTCTGACCAGCAGCCTATGAG 3300
GGGGTTTGGCACCAAGCTCTGTCCAATCAGGTAGGCTGGGCCTGAACTAGCC 3352
AATCAGATCAACTCTGTCTTGGGCGTTTGAACTCAGGGAGGGAGGCCCTTGG 3404
GAGCAGGTGCTTGTGGACAAGGCTCCACAAGCGTTGAGCCTTGGAAAGGTAG 3456
ACAAGCGTTGAGCCACTAAGCAGAGGACCTTGGGTTCCCAATACAAAAATAC 3508
CTACTGCTGAGAGGGCTGCTGACCATTTGGTCAGGATTCCTGTTGCCTTTAT 3560
ATCCAAAATAAACTCCCCTTTCTTGAGGTTGTCTGAGTCTTGGGTCTATGCC 3612
TTGAAAAAAGCTGAATTATTGGACAGTCTCACCTCCTGCCATAGGGTCCTGA 3664
ATGTTTCAGACCACAAGGGGCTCCACACCTTTGCTGTGTGTTCTGGGGCAAC 3716

Fig. 1b cont'd

CTACTAATCCTCTCTGCAAGTCGGTCTCCTTATCCCCCCAAATGGAAATT 3766

GTATTTGCCTTCTCCACTTTGGGAGGCTCCCACTTCTTGGGAGGGTTACA 3816

TTTTTTAAGTCTTAATCATTTGTGACATATGTATCTATACATCCGTATCTT 3867

TTAATGATCCGTGTGTACCATCTTTGTGATTATTTCCTTAATATTTTTTCT 3918

TTAAGTCAGTTCATTTTCGTTGAAATACATTTATAAAGAAAAATCTTTGTT 3969

ACTCTGTAAATGAAAAAACCCATTTTCGCTATAAATAAAAGGTAACTGTAC 4020

AAAATAAGTACAAT 4034

Neu eingereicht / Newly filed
Nouvellement déposé

FIG. 2

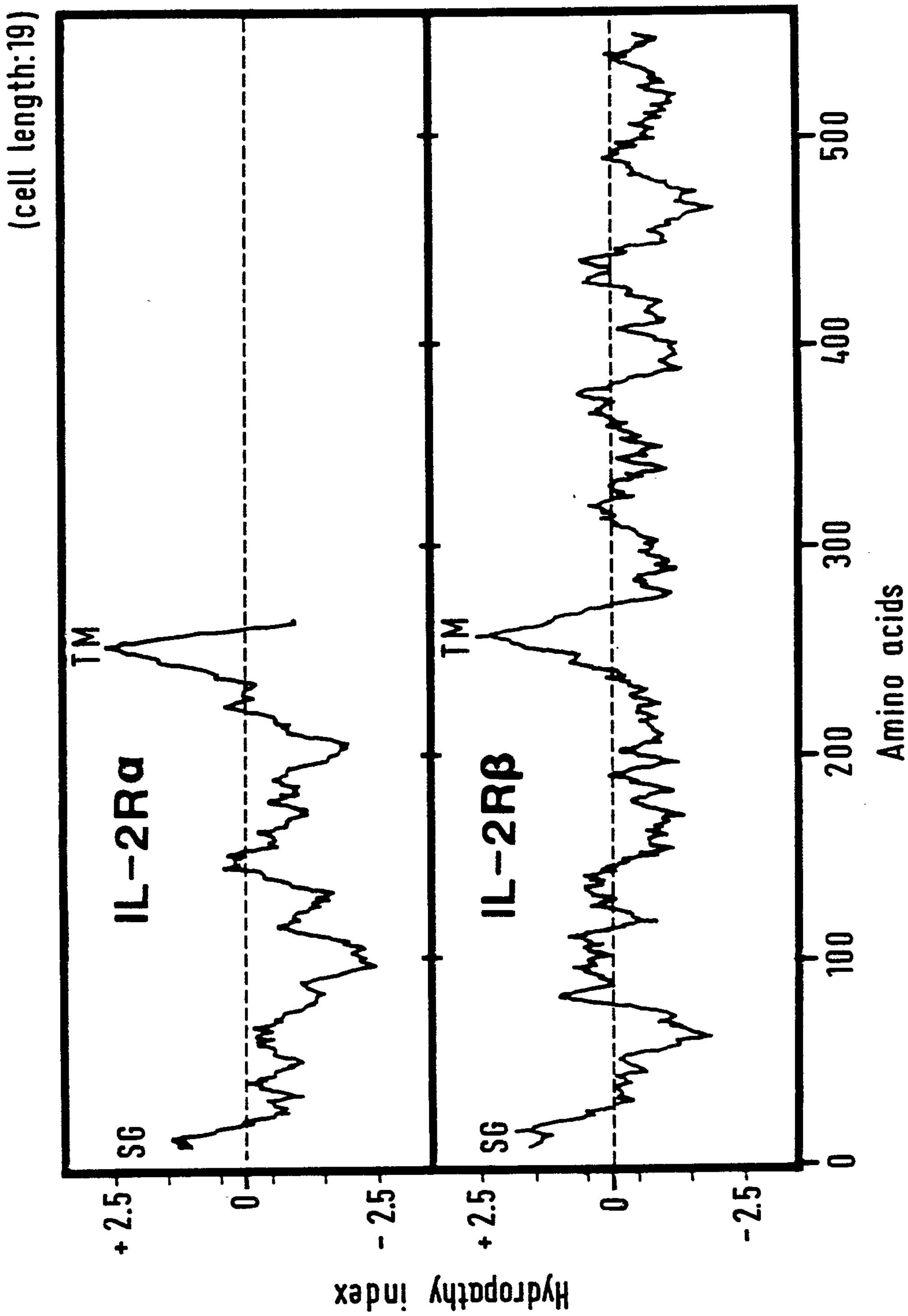

Neu eingereicht / Newly filed
Nouvellement déposé

FIG. 3a

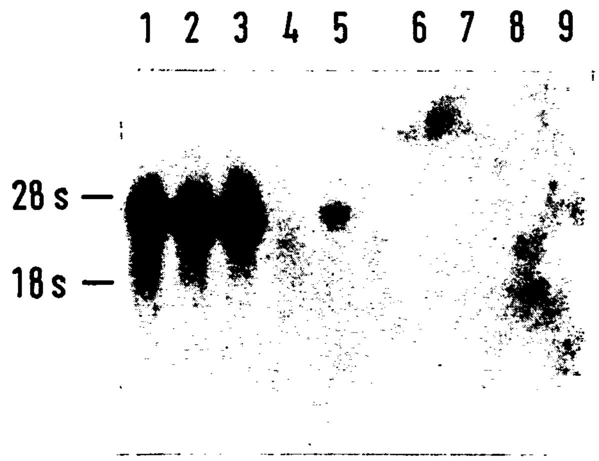


FIG. 3b

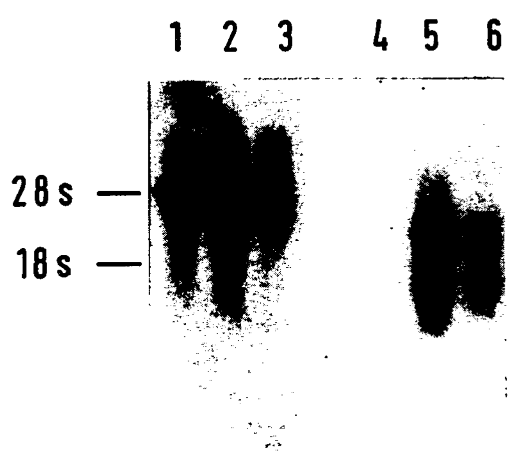

Neu eingereicht / Newly filed
Nouvellement déposé

FIG. 4a

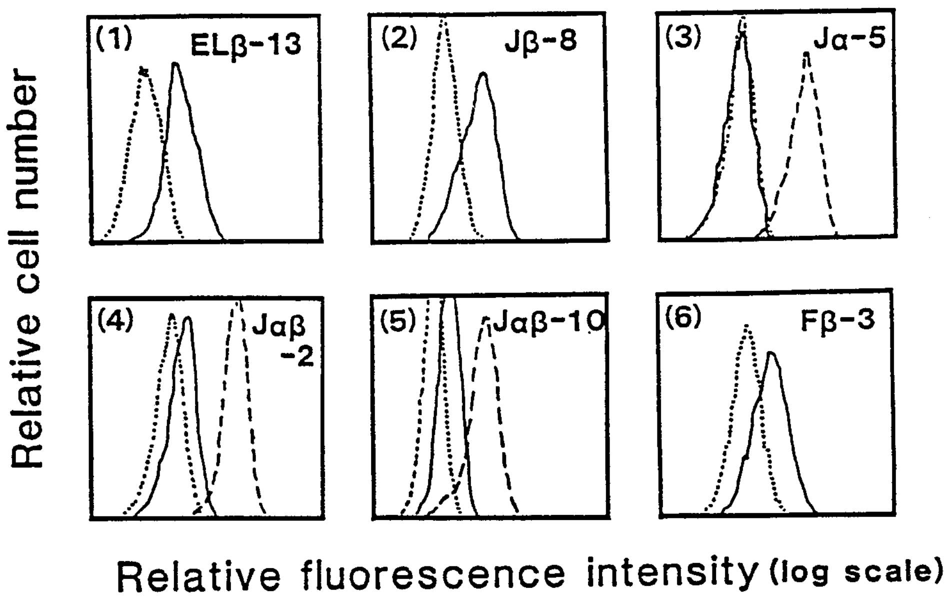


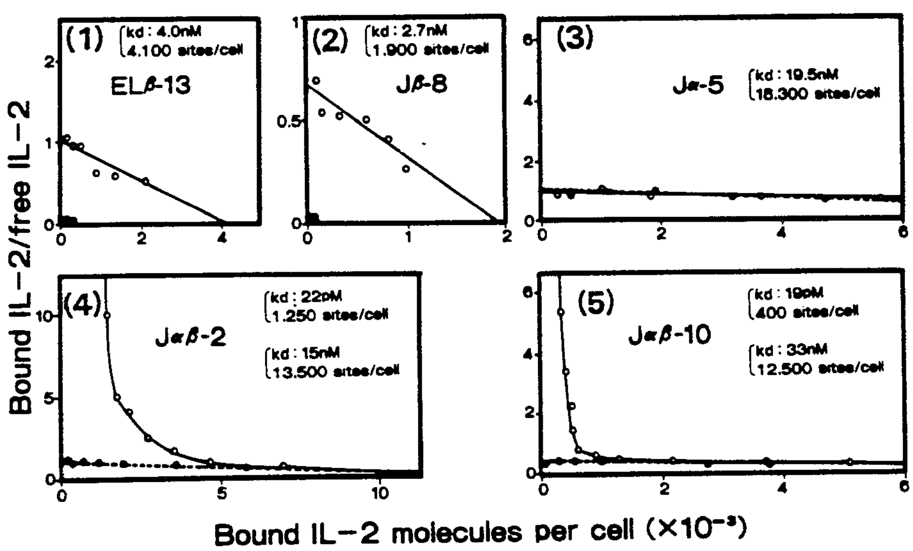


FIG. 4b

Neu eingereicht / Newly filed
Nouvellement déposé

FIG. 5

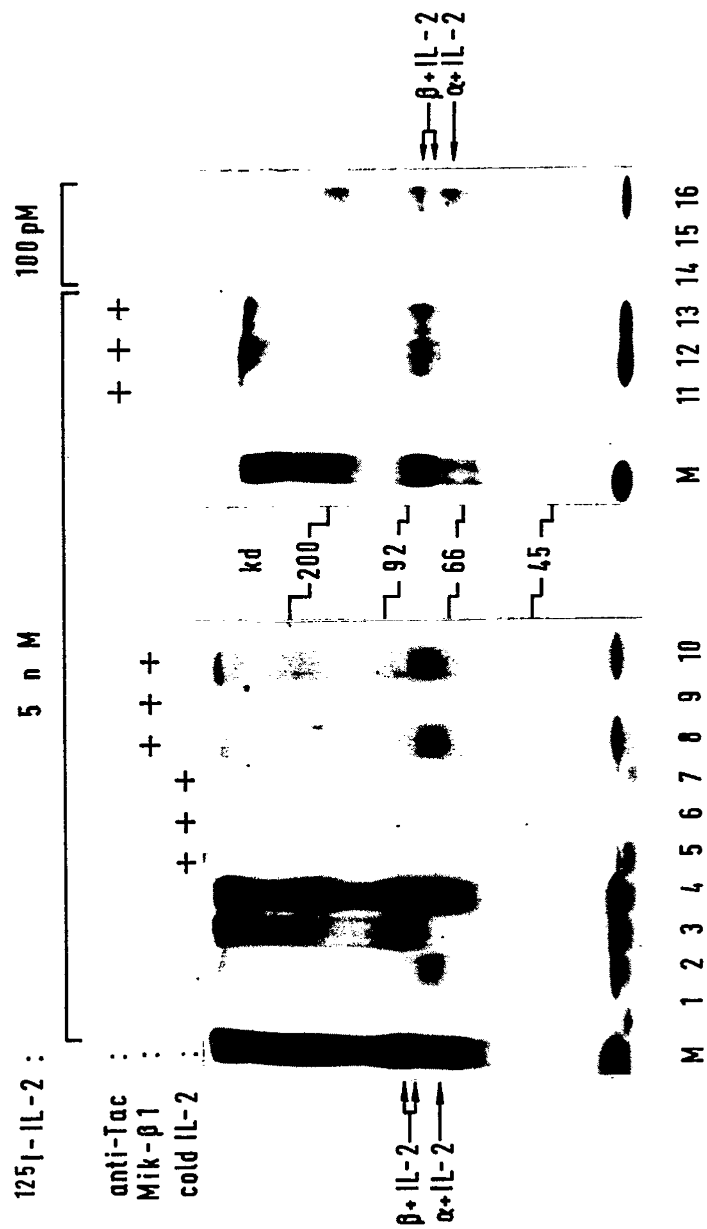

Neu eingereicht / Newly filed
Nouvellement déposé

FIG. 6

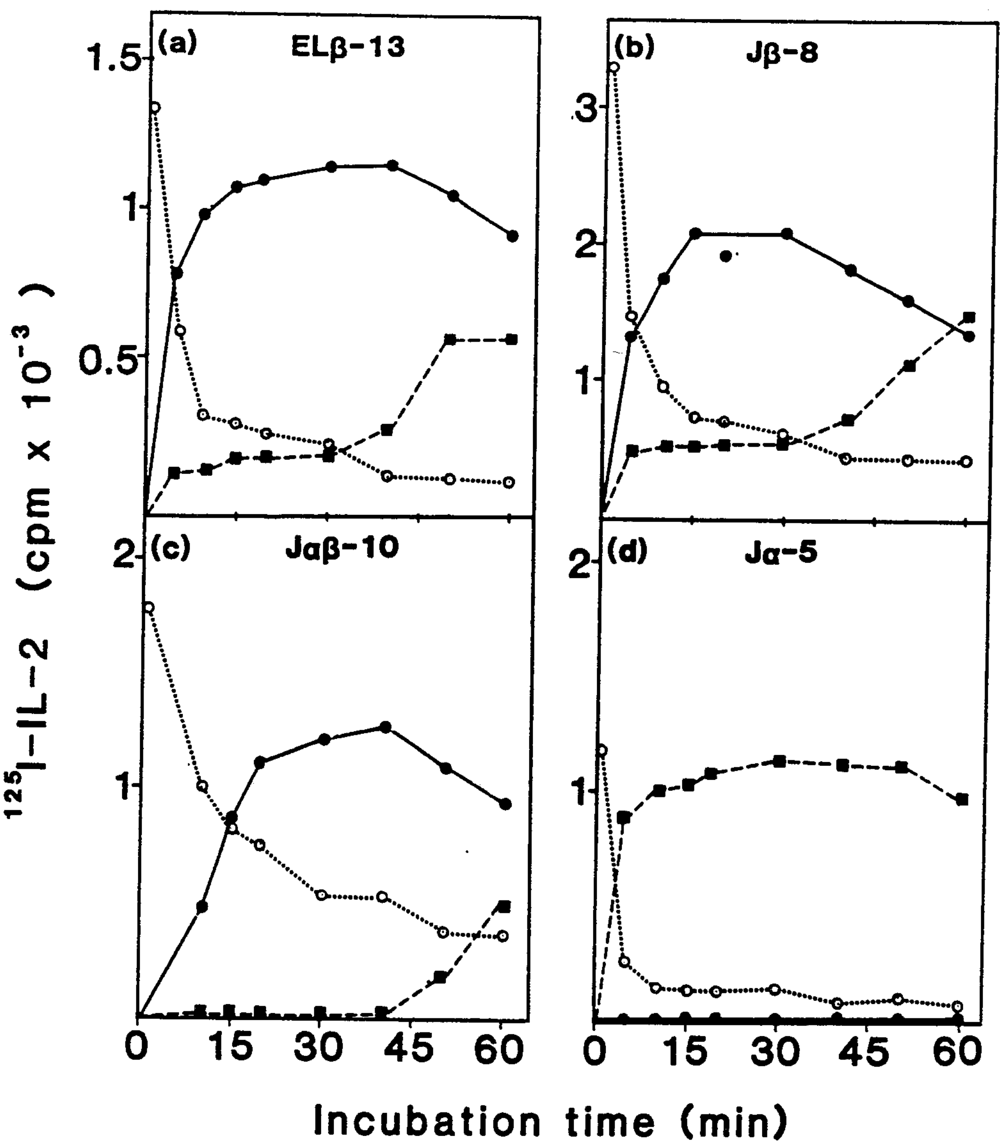

European Patent Office

# EUROPEAN SEARCH REPORT

Application Number

EP 89 10 4023

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X<br><br><br>Y | WO-A-8 900 168 (THE UNITED STATES OF AMERICA)<br>* Page 6, line 1 - page 8, line 30 *<br>--- | 9,10<br><br><br>1-8 | C 12 N 15/00<br>C 12 P 21/02<br>C 12 N 5/00<br>C 12 P 21/00 //<br>(C 12 P 21/00<br>C 12 R 1:91 ) |
| Y | EP-A-0 162 699 (IMMUNEX CORP.)<br>--- | 1-8 | |
| T | SCIENCE, vol. 244, 5th May 1989, pages 551-556, Washington, DC, US; M. HATAKEYAMA et al.: "Interleukin-2 receptor beta chain gene: generation of three receptor forms by cloned human alpha and beta chain cDNA's"<br>* Whole document *<br>--- | 1-10 | |
| T,D | PROCEEDINGS OF THE NATIONAL ACADEMY OF SCIENCE, USA, vol. 86, March 1989, pages 1982-1986, Washington, DC, US; M. TSUDO et al.: "Characterization of the interleukin 2 receptor beta chain using three distinct monoclonal antibodies"<br>* Page 1982: "Materials and methods" *<br>--- | 9,10 | TECHNICAL FIELDS SEARCHED (Int. Cl. 5) |
| A | JOURNAL OF EXPERIMENTAL MEDICINE, vol. 165, January 1987, pages 223-238, The Rockefeller University Press, New York, US; K. TESHIGAWARA et al.: "Interleukin 2 high-affinity receptor expression requires two distinct binding proteins"<br>* Page 223, lines 6-9 *<br>----- | | C 12 N<br>C 12 P |

~~The present search report has been drawn up for all claims~~

| Place of search | Date of completion of the search | Examiner |
|---|---|---|
| THE HAGUE | 21-08-1989 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent
Office

## ☒ CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☒ No claims fees have been paid within the prescribed time limit. The present European search report has been drawn up for the first ten claims.

## ☐ LACK OF UNITY OF INVENTION

The Search Division considers that the present European patent application does not comply with the requirement of unity of invention and relates to several inventions or groups of inventions,
namely:

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☐ None of the further search fees has been paid within the fixed time limit. The present European search report has been drawn up for those parts of the European patent application which relate to the invention first mentioned in the claims,

namely claims: